(19) Europäisches Patentamt
European Patent Office
Office européen des brevets

(11) Publication number: 0 207 497 A2

(12) EUROPEAN PATENT APPLICATION

(21) Application number: 86108949.8

(22) Date of filing: 01.07.86

(51) Int. Cl.[4]: C07D 401/04 , A61K 31/47

(30) Priority: 03.07.85 JP 146413/85

(43) Date of publication of application: 07.01.87 Bulletin 87/02

(84) Designated Contracting States: AT BE CH DE FR GB IT LI LU NL SE

(71) Applicant: KYORIN PHARMACEUTICAL CO., LTD.
No. 5, Kanda Surugadai 2-chome
Chiyoda-ku Tokyo(JP)

(72) Inventor: Masuzawa, Kuniyoshi
No. 5-71, Nishi-cho
Koga-shi Ibaragi-ken(JP)
Inventor: Suzue, Seigo
No. 13-4, Aoba 4-chome
Kuki-shi Saitama-ken(JP)
Inventor: Hirai, Keiji
No. 1-2-512, Aoba 1-chome
Kuki-shi Saitama-ken(JP)
Inventor: Ishizaki, Takayoshi
No. 9-6, Sakurada 4-chome Washimiya-machi
Kitakatsushika-gun Saitama-ken(JP)

(74) Representative: Patentanwälte TER MEER - MÜLLER - STEINMEISTER
Mauerkircherstrasse 45
D-8000 München 80(DE)

# (54) Quinolonecarboxylic acid derivative and process for its preparation.

(57) Quinolonecarboxylic acid derivative of the following formula;

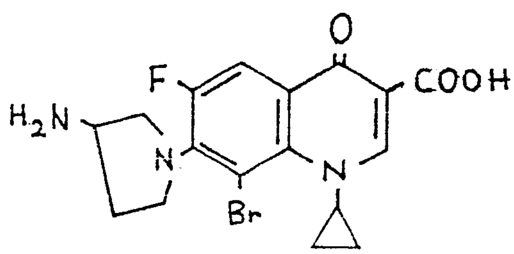

hydrates and pharmaceutically acceptable salts thereof are useful as an antibacterial agent.

EP 0 207 497 A2

# Quinolonecarboxylic acid derivative and process for its preparation

Detailed description of the invention:

The present invention relates to a novel quinolonecarboxylic acid derivative of the following formula (I),

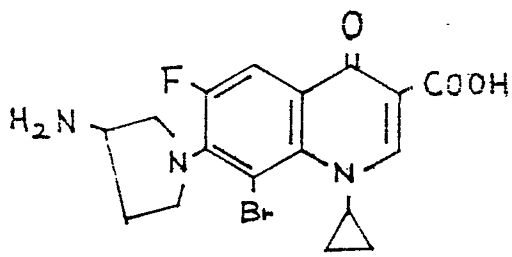

(I)

its hydrates and salts, preparation process therefor and uses thereof as an antibacterial agent.

The compound represented by the formula (I), contains optical isomers, owing to an asymmetric carbon on the aminopyrrolidine ring, the 7-substituent. But all of optical isomers and their mixture are represented, for convenience, by the unitary formula. Hence, the scope of the invention is not limited to one of the optical isomers or their mixture.

Quinolonecarboxylic acid antibacterial agent began with nalidixic acid and have been developed to piromidic acid, furthermore, to pipemidic acid. They are useful agents for the medical treatment against urinary tract infections with aerobic gram-negative bacteria.

Norfloxacin, which has been developed by us recently, exhibits potent antibacterial activity not only against gram-negative bacteria but also against gram-positive bacteria. Moreover, its potency is extremely stronger than the previous quinolonecarboxylic acids. Norfloxacin became an epoch-making progress in this field, it has been used clinically very often at the present time.

The later quinolonecarboxylic acids, such as, ofloxacin and ciprofloxacin, which have substituents similar to norfloxacin, have been continuously developed.

Ciprofloxacin possesses stronger antibacterial activity than that of norfloxacin. But the antibacterial potency against gram-positive bacteria is fairly inferior to that against gram-negative bacteria.

On the other hand, the increase of β-lactam antibiotics-resistant gram-positive bacteria such as methicillin and cephalosporin resistant Staphylococcus aureus, S. epidermidis, Enterococcus faecalis, have made a trouble in clinical field.

In addition, it has been evidenced that obligate anaerobes on skin or mucous membrane act as the pathogen of opportunistic infection owing to the popularization of anaerobes inspection as a result of the development of clinical testing technology. It has been reported that anaerobes are found with or without an aerobic bacteria at the rate of 50-80 % on respiratory tract infections, intraperitoneal infections, chronic otitis media, paranasal sinusitis and obstetrical and gynecological infections, and the rate of the combination of anaerobes with Escherichia coli, Enterococcus faecalis and the other Streptococci is about 95 %. Gradually increasing of the resistance rate of anaerobes to β-lactam antibiotics or clindamycin, which were inherently effective against anaerobes, caused serious problem for choice of chemotherapeutic agents.

On these background, the development of new antimicrobial agents with stronger activity and broader spectrum is required.

It was found that, in animals, the compound of the invention showed good oral absorption, excellent tissue distribution, satisfying biological stability and acceptability.

In following, explanation is made about the preparation process for the compound of the invention.

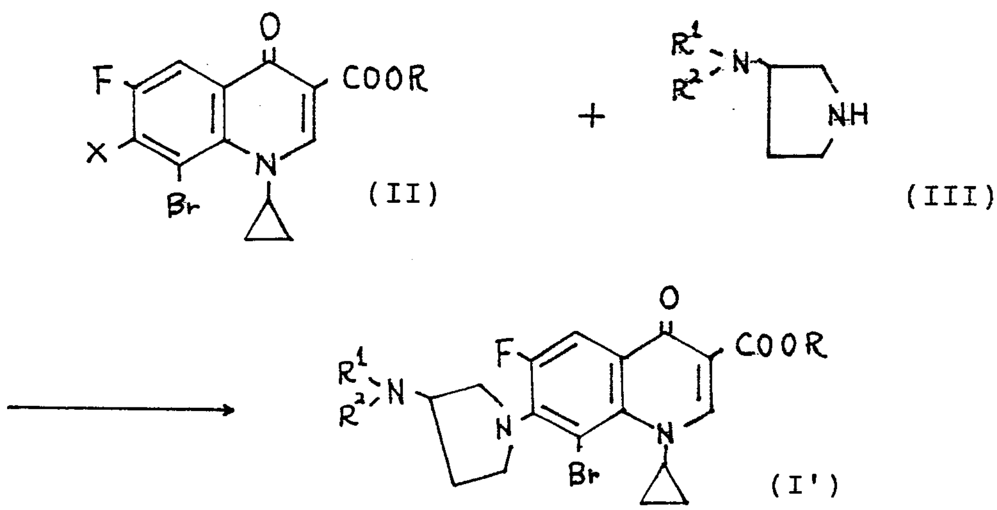

(II) (III) (I')

wherein R is a hydrogen atom or a lower alkyl group, $R^1$ and $R^2$ are each independently a hydrogen atom, a lower acyl group, a lower alkoxycarbonyl group, a benzyl group which may be substituted, a trityl group and an aliphatic or aromatic sulfonyl group and X is a halogen atom.

Namely, by allowing compounds represented by the formula (II) to react with amines represented by the formula (III), compound of the invention represented by the formula (I') is synthesized. The reaction of compounds represented by the formula (II) with compounds represented by the formula - (III) preferably is carried out by heating the mixture in a solvent such as water, alcohols, acetonitrile, dimethylformamide (DMF), dimethyl sulfoxide - (DMSO), hexamethylphosphoric triamide, pyridine, picoline and the like or in the absence of the solvent. The reaction temperature is selected appropriately in a range of room temperature to 200 °C, preferably room temperature to 160 °C. In more details, it is preferable to allow compounds represented by the formula (II) to react with 1 to 5 times mole of compounds represented by the formula (III) for 1 to several hours at room temperature to 120 °C in 2 to 10 times volume of aforementioned solvents. At this time, the use of deacidifying agents such as triethylamine, diazabicyclo bases and potassium carbonate is also desirable.

Moreover, compounds (I') wherein $R^1$ or $R^2$ is a lower acyl group, a lower alkoxycarbonyl group, a benzyl group which may be substituted, a trityl group, can be converted to amino group according the usual method to offer the compound of the invention, for example, hydrolysis with acid or alkali, catalytic reduction and so on.

Moreover, compounds (I') wherein R is a lower alkyl group can be hydrolyzed according to the usual method and ester is converted to carboxylic acid to offer the compound of the invention. Such hydrolysis can be carried out easily with alkalies such as potassium hydoxide or acids such as sulfuric acid at room temperature to boiling point of solvents in water, mixed liquor of water with alcohols, mixed liquor of water with acetic acid, and so on.

Furthermore, the compounds of the formula (I) can be converted, if desired, to the pharmaceutically acceptable ammonium salts or carboxylic acid metal salts by treatment with acid or alkali. The acid may be organic or inorganic acids such as, for example, hydrochloric acid, sulfuric acid, phosphoric acid, acetic acid, methanesulfonic acid, oxalic acid and lactic acid. The carboxylic acid metal salts may be, for example, sodium, potassium, magnesium, calcium, aluminum, cerium, chromium, cobalt, copper, iron, zinc, platinum and silver salts.

The compound of the formula (I), hydrates and salts thereof may be used as medicines in the conventional form of pharmaceutical preparations, which may be, for example, tablets, capsules, powder, ointments, suppositories, injections or eye drops, suitable for peroral, parental, enteral or local administration.

The following examples will further illustrate the present invention without, however, limiting it thereto.

Example 1 7-(3-Amino-1-pyrrolidinyl)-8-bromo-1-cyclopropyl-6-fluoro-1,4-dihydro-4-oxo-3-quinolinecarboxylic acid

A mixture of 8-bromo-1-cyclopropyl-6,7-difluoro-1,4-dihydro-4-oxo-3-quinolinecarboxylic acid (200 mg), anhydrous acetonitrile (3 ml), 1,8-diazabicyclo[5,4,0]-7-undecene (95 mg) and 3-t-butoxycarbonylaminopyrrolidine (280 mg) was refluxed for an hour under stirring. The reaction mixture was stirred for further 4 hours at room temperature and then concentrated. To the resulting residue was added the cooled mixture of concentrated hydrochloric acid-methanol (4 ml; 1:1), stirred for 45 minutes in an ice bath. The reaction mixture was neutralized with concentrated aqueous

ammonia, the resulting precipitate was collected by filtration, washed with water and recrystallized from chloroformmethanol to give the title compound (80 mg) as pale yellow prisms, mp 207-210 °C (decompd.).

Analysis (%) for $C_{17}H_{17}BrFN_3O_3 \bullet 2H_2O$, Calcd. - (Found): C, 45.75 (45.88); H, 4.74 (4.25); N, 9.42 - (9.42).

Experiment 1. Antibacterial spectrum

Minimal inhibitory concentrations (MICs) were determined in accordance with the method recommended by Japan Society of Chemotherapy. The results are shown in Table 1.

Table 1-1 In vitro antibacterial activity (standard strains)

| Organism ($10^6$ cells/ml) | Gram | MIC (μg/ml) | |
|---|---|---|---|
| | | Exp. 1 | CPFX |
| Bacillus subtilis PCI 219 | + | 0.025 | 0.05 |
| Staphylococcus aureus 209 P | + | 0.05 | 0.20 |
| S. aureus Smith | + | 0.05 | 0.39 |
| S. aureus IID 670 (Terajima) | + | 0.05 | 0.20 |
| S. epidermidis IID 866 | + | 0.10 | 0.20 |
| Streptococcus pyogenes (S-8) | + | 0.10 | 0.39 |
| S. pyogenes IID 692 | + | 0.20 | 0.78 |
| S. pneumoniae IID 552 | + | 0.10 | 0.78 |
| E. faecalis IID 682 | + | 0.10 | 0.78 |
| Escherichia coli NIHJ JC-2 | - | 0.0063 | 0.0063 |
| E. coli ATCC 10536 | - | 0.0125 | 0.0125 |
| E. coli ML 4707 | - | 0.0125 | 0.0125 |
| Proteus vulgaris IFO 3167 | - | 0.0125 | 0.0125 |
| P. mirabilis IID 994 | - | 0.0125 | 0.0125 |
| Morganella morganii IID 602 | - | 0.05 | 0.025 |
| Klebsiella pneumoniae KY(GN)6445 | - | 0.0125 | 0.0125 |
| K. pneumoniae 1-220S | - | 0.025 | 0.025 |
| Enterobacter cloacae IID 977 | - | 0.025 | 0.025 |
| Citrobacter freundii IID 976 | - | 0.025 | 0.0063 |
| Serratia marcescens IID 618 | - | 0.05 | 0.025 |
| Shigella sonnei IID 969 | - | 0.0063 | 0.0063 |
| Salmonella enteritidis IID 604 | - | 0.025 | 0.025 |
| Pseudomonas aeruginosa V-1 | - | 0.20 | 0.05 |
| P. aeruginosa IFO 12689 | - | 0.39 | 0.20 |
| P. aeruginosa IID 1210 | - | 0.78 | 0.78 |
| P. cepacia GIFU 518 | - | 0.39 | 0.39 |
| P. maltophilia GIFU 2491 | - | 0.05 | 0.39 |

Table 1-2 In vitro antibacterial activity (standard strains)

| Organism ($10^6$ cells/ml) | Gram | MIC (μg/ml) | |
|---|---|---|---|
| | | Exp. 1 | CPFX |
| Yersinia enterocolitica IID 981 | - | 0.025 | 0.025 |
| Acinetobacter anitratus IID 876 | - | 0.05 | 0.10 |
| Alcaligenes faecalis 0114002 | - | 0.10 | 0.39 |
| Bacteroides fragilis GM 7000 | - | 0.10 | 6.25 |
| B. fragilis 0558 | - | 0.10 | 3.13 |
| B. fragilis 25285 | - | 0.10 | 3.13 |
| B. distasonis 8503 | - | 0.39 | 6.25 |
| B. thetaiotaomicron (0661) | - | 0.20 | >12.5 |
| B. vulgatus KYA 29327 | - | 0.20 | >12.5 |
| Fusobacterium mortiferum 4249 | - | 0.20 | 1.56 |
| F. necrophorum S-45 | - | 0.20 | 0.78 |
| F. varium KYA 8501 | - | 0.78 | >12.5 |
| Eubacterium lentum GAI 5242 | + | 0.10 | 0.78 |
| Propionibacterium acens 11828 | + | 1.56 | 12.5 |
| Peptococcus magnus KY 017 | + | 0.10 | 0.39 |
| Clostridium difficile I-E | + | 0.78 | 12.5 |
| C. perfringens KYA 13123 | + | 0.20 | 0.39 |
| C. ramosum | + | 1.56 | 12.5 |
| Peptostreptococcus anaerobius KYA 27337 | + | 0.20 | 1.56 |
| Pst. micros UPI 5464-1 | + | 0.10 | 0.20 |
| Veillonella parvula KYA 10790 | - | 0.10 | 0.20 |

CPFX : ciprofloxacin

**Claims**

Claims for the designated states: DE, FR, GB, IT, BE, NI, CH, SE, LU,

1. A compound of the formula (I);

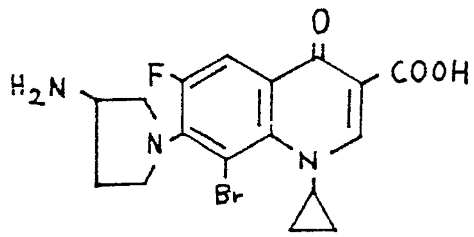

(I)

the hydrates or the pharmaceutically acceptable acid addition or alkali salts thereof.

2. A process for the preparation of a compound of the formula (I),

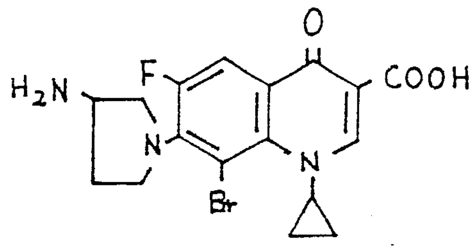

(I)

which comprises condensing a compound of the formula (II);

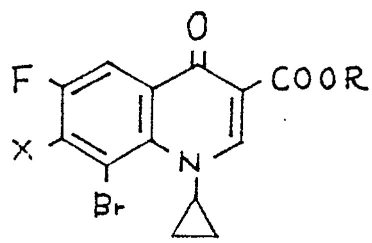

(II)

[wherein R is a hydrogen atom or a lower alkyl group, X is a halogen atom], with a compound of the formula (III);

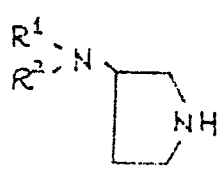

(III)

[wherein $R^1$ and $R^2$ are each independently a hydrogen atom, a lower acyl group, a lower alkoxycarbonyl group, a benzyl group which may be substituted, a trityl group and an aliphatic or aromatic sulfonyl group]; however in which, when both or either of $R^1$ and $R^2$ are a lower acyl group, a lower alkoxycarbonyl group, a benzyl group which may be substituted, a trityl group and an aliphatic or aromatic sulfonyl group, the condensed product is followed by removal of the protecting group to an amino derivative (I) and when R is a lower alkyl group, the condensed product is followed by hydrolysis to the carboxylic acid derivative (I).

3. An antibacterial pharmaceutical composition containing the compound according to claim 1 and an inert pharmaceutical acceptable carrier.

Claims for the designated state: AT

1. A process for the preparation of a compound of the formula (I),

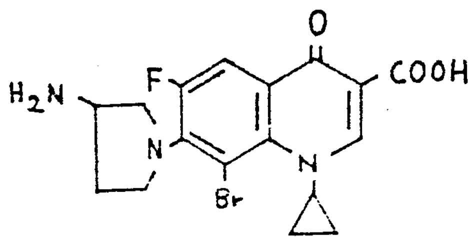

(I)

and the hydrates or the pharmaceutically acceptable acid addition or alkali salts thereof, which comprises condensing a compound of the formula (II);

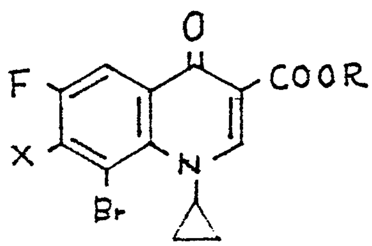

(II)

wherein R is a hydrogen atom or a lower alkyl group, X is a halogen atom/, with a compound of the formula (III);

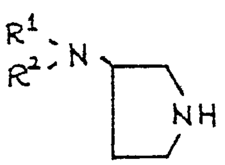

(III)

wherein $R^1$ and $R^2$ are each independently a hydrogen atom, a lower acyl group, a lower alkoxycarbonyl group, a benzyl group which may be substituted, a trityl group and an aliphatic or aromatic sulfonyl group/; however in which, when both or either of $R^1$ and $R^2$ are a lower acyl group, a lower alkoxycarbonyl group, a benzyl group which may be substituted, a trityl group and an aliphatic or aromatic sulfonyl group, the condensed product is followed by removal of the protecting group to an amino derivative (I) and when R is a lower alkyl group, the condensed product is followed by hydrolysis to the carboxylic acid derivative (I).